Europäisches Patentamt

European Patent Office

Office européen des brevets

(11) Numéro de publication: **0 309 345 B1**

# FASCICULE DE BREVET EUROPEEN

(45) Date de publication de fascicule du brevet: **22.09.93** (51) Int. Cl.5: **A61L 33/00**

(21) Numéro de dépôt: **88402380.5**

(22) Date de dépôt: **21.09.88**

(54) **Article en matériau polymère, modifié en surface pour présenter une hémocompatibilité améliorée et une thrombogénéicité diminuée, et son procédé d'obtention.**

(30) Priorité: **23.09.87 FR 8713154**

(43) Date de publication de la demande:
**29.03.89 Bulletin 89/13**

(45) Mention de la délivrance du brevet:
**22.09.93 Bulletin 93/38**

(84) Etats contractants désignés:
**AT BE CH DE ES GB GR IT LI LU NL SE**

(56) Documents cités:
**EP-A- 0 096 573**
**DE-A- 3 116 026**
**FR-A- 2 446 682**
**US-A- 3 740 325**
**US-A- 4 091 166**

**CHEMICAL ABSTRACTS, vol. 96, no. 2, 11 janvier 1982, page 332, no. 11635u, Columbus, Ohio, US; Y. ISHIKAWA et al.: "Surface structure of glow discharge-treated poly(vinyl chloride) and the interaction with platelets"**

(73) Titulaire: **INSTITUT DE RECHERCHE APPLI-OUEE SUR LES POLYMERES (I.R.A.P.)
Route de Laval
F-72000 Le Mans(FR)**

(72) Inventeur: **Legeay, Gilbert
12 rue de l'Eglise Saint Saturnin
F-72650 La Milesse(FR)**
Inventeur: **Gardette, Jacques
8 rue Léonard de Vinci
F-77330 Ozoir La Ferrière(FR)**
Inventeur: **Brosse, Jean-Claude
La Closeraie Duneau
F-72160 Connerre(FR)**
Inventeur: **de la Faye, Dominique
10 rue Alain Fournier
F-29200 Brest(FR)**
Inventeur: **Legendre, Jean-Michel
11 rue Loucheur
F-29200 Brest(FR)**

(74) Mandataire: **Phélip, Bruno et al
c/o Cabinet Harlé & Phélip 21, rue de La Rochefoucauld
F-75009 Paris (FR)**

# EP 0 309 345 B1

**Description**

La présente invention concerne, dans le domaine médical et de l'analyse médicale, la fabrication d'articles destinés à être utilisés dans des applications nécessitant qu'ils présentent une forte hémocompatibilité et une faible thrombogénéicité.

Comme articles de ce genre, on peut mentionner les objets implantables, comme les prothèses vasculaires, les prothèses valvulaires cardiaques et les cathéters de tous types, et, d'une façon générale, tout matériel destiné à venir en contact avec le sang ou les tissus humains, à l'intérieur du corps humain comme à l'extérieur, par exemple, le matériel de laboratoire d'analyse sanguine.

Les objets implantables généralement utilisés à l'heure actuelle sont réalisés en divers matériaux polymères, parmi lesquels on peut mentionner les polymères acryliques, les résines époxy, les silicones, le polypropylène, les polymères fluorés et les polycarbonates. Quant aux objets à usage externe, ils peuvent être constitués par l'un des matériaux polymères précités et, également, être en caoutchouc, résine acétal, polyuréthanne, poly(chlorure de vinyle).

Dans le cas des prothèses cardiovasculaires, on utilise toutefois actuellement principalement deux types de matériaux, à savoir le polytétrafluoréthylène (par exemple, celui commercialisé sous la dénomination 《TEFLON》), et le poly (éthylène téréphtalate) (par exemple, commercialisé sous la dénomination 《DACRON》), par exemple sous forme tissée notamment avec un fil simple, non crêpé ; sous forme tricotée, souvent avec un fil crêpé, le cas échéant avec réalisation de petites boucles à l'intérieur et à l'extérieur de l'objet, afin de donner du confort, ce qui est désigné par l'appellation 《tricoté double velours greffé》.

Les prothèses vasculaires en éléments tricotés sont actuellement les plus utilisées car elles offrent des qualités mécaniques intéressantes, et, en particulier, un grand confort lors de l'implantation. Toutefois, comme de telles prothèses sont poreuses, on peut utiliser à la place des prothèses tissées présentant une porosité beaucoup plus faible, auquel cas elles sont beaucoup moins souples. Le choix dépend donc de l'application particulière envisagée. Ainsi, l'emploi du polytétrafluoréthylène est limité au cas des prothèses vasculaires de petits diamètres, (de l'ordre du millimètre), car il ne présente pas les qualités mécaniques souhaitables pour les prothèses cardiovasculaires de gros diamètres.

La plupart de ces biomatériaux présentent les inconvénients, d'une part, d'un caractère hydrophile important les rendant perméables à l'eau, au plasma et au sang, la perméabilité pouvant également être due à leur texture, et, d'autre part, d'un caractère thrombogène et d'une aptitude à la fixation de globules rouges sur les parois des implants, soit seuls, soit en amas, pouvant entraîner un ralentissement du flux sanguin et une obstruction partielle ou totale du vaisseau.

Ces inconvénients nécessitent de réaliser, avec les prothèses cardiovasculaires, une phase de précoagulation quelques minutes avant l'implantation.

Afin d'éviter cette opération à caractère dangereux, divers palliatifs ont été expérimentés, et notamment la fixation de collagène, d'albumine ou de gélatine à la surface d'objets en poly(éthylène téréphtalate) ou le traitement au glutaraldéhyde de telles surfaces. Ainsi traitées, ces prothèses deviennent non poreuses.

Cependant, il s'avère que ces techniques ne sont pas complètement satisfaisantes, aussi bien pour le clinicien que pour le patient.

Dans le même but, on a par ailleurs proposé de modifier la surface des matériaux polymères par traitement par un plasma ou par polymérisation par plasma (H. Yasuda et M. Gaziki, Biomaterials, 68, 3, 1982). C'est ainsi qu'on a proposé de traiter un matériau polymère par un plasma d'hydrogène et d'azote ou d'ammoniac, de façon à introduire des groupes amino capables de fixer ensuite de l'héparine. Ce traitement, s'il retarde la coagulation, ne permet pas de l'empêcher. Quant à la polymérisation par plasma de monomères tels que l'éthylène ou le tétrafluoréthylène, elle conduit à la formation, à la surface des matériaux, d'une pellicule irrégulière qui n'est pas suffisamment adhérente et qui finalement se décolle.

Egalement, est proposé, selon la demande de brevet allemand n° 3 116 026 un procédé de fabrication d'une couche biocompatible sur des prothèses en contact avec le sang. Ce procédé consiste à effectuer une décharge par plasma à basse pression, le gaz réactionnel pouvant être le tétrafluorométhane. Cependant, celui-ci n'est mentionné que pour le traitement du polytétrafluoréthylène.

La demande de brevet français n° 2 446 682 décrit un procédé pour le traitement des surfaces internes d'un corps tubulaire en matière plastique au moyen d'un plasma à basse température. Pour un tel traitement, il est indiqué que l'on peut utiliser des gaz de nature organique, ceux-ci étant recommandés lorsque l'amélioration de l'adaptation à l'organisme vivant est le but recherché. Le seul exemple mettant en jeu un gaz organique pour le plasma est l'exemple 5, ledit gaz étant hexaméthyldisiloxane et le traitement effectué ayant pour résultat que la surface interne de l'objet tubulaire est revêtue d'un film mince, conduisant donc aux inconvénients évoqués ci-dessus. Par ailleurs, le traitement décrit dans cette demande

2

de brevet français s'effectue dans une huile isolante. Une telle méthode est difficilement applicable à des biomatériaux, car il faut laver les objets puis les sécher, et elle est inapplicable à des prothèses vasculaires, lesquelles, étant poreuses, ne tiennent pas au vide, du moins si celui-ci n'est appliqué qu'à l'intérieur de l'élément tubulaire, comme c'est le cas de ce traitement de la technique antérieure. En outre, un tel traitement est un traitement en continu qui ne s'applique qu'à des objets réguliers constitués d'un simple tube, et ne permet pas de traiter des objets sans géométrie particulière, c'est-à-dire aussi bien tubulaires qu'ayant des formes spéciales, comportant par exemple des orifices, des étranglements, des formes en Y, pour des cathéters par exemple.

La présente invention a pour but de remédier à l'ensemble de ces inconvénients ; elle propose à cet effet un article en un matériau polymère qui présente une hémocompatibilité améliorée et une thrombogénéicité diminuée, les propriétés mécaniques du matériau polymère de base et la tolérance par l'organisme et/ou la biocompatibilité dudit matériau n'étant pas altérées.

Selon l'invention, on propose un matériau polymère contenant des atomes d'hydrogène, qui est modifié en surface, dans sa structure chimique, de façon spécifique, une telle modification de structure chimique étant fondamentalement différente d'un film rapporté sur la surface de l'objet, lequel se trouve nécessairement lié de façon précaire à cette surface, en apportant une discontinuité dans la structure et le risque de délaminage déjà évoqué lors de sollicitations mécaniques ou lors du passage de fluides (eau, sang, etc. ). Ainsi, cette modification spécifique, non évidente en soi pour l'homme du métier, permet au matériau de base de garder sa texture et ses propriétés mécaniques pour lesquelles il a été choisi dans l'application particulière envisagée.

La présente invention a donc d'abord pour objet un article destiné à être utilisé dans des applications nécessitant qu'il présente une forte hémocompatibilité et une faible thrombogénéicité, au moins sur la partie de sa surface (dite surface exposée) susceptible de venir en contact avec du sang, ledit article étant constitué d'un matériau polymère d'origine naturelle ou synthétique contenant des atomes d'hydrogène, caractérisé par le fait que des atomes d'hydrogène présents sur sa surface exposée ont été remplacés par des atomes de fluor ou des groupements $CF_2$ ou $CF_3$, de telle sorte que le fluor total représente au plus 10% des atomes présents sur cette surface, tel que peut l'indiquer la spectroscopie dite ESCA.

On peut notamment citer les objets venus du moulage ou de l'extrusion pour constituer notamment des cathéters, qui peuvent par exemple être en polyéthylène, des seringues qui peuvent être par exemple en polyéthylène ou en poly(chlorure de vinyle), ou similaires ; ainsi que les objets réalisés en matériaux tissés ou tricotés pour constituer notamment des prothèses vasculaires ou des prothèses valvulaires cardiaques, réalisés par exemple en poly(éthylène téréphtalate).

Comme autres polymères utilisables selon la présente invention, on peut mentionner, entre autres, les polyuréthannes, le poly(chlorure de vinyle), les silicones, le polystyrène et les polyacrylates.

Le procédé d'obtention de l'article qui vient d'être défini consiste à effectuer un traitement de surface dudit article se trouvant dans la forme appropriée pour son utilisation, par un plasma de molécules fluorées non polymérisables, dans des conditions ménagées.

Certes, on a déjà proposé, conformément au brevet des Etats-Unis d'Amérique n° 3 740 325, un procédé de traitement de matériaux polymères tels que des polyoléfines et des polyesters, ce procédé consistant à effectuer un traitement avec des gaz fluorés, sous l'action d'une décharge électrique de façon à remplacer les atomes d'hydrogène par les atomes de fluor. Ce procédé est décrit pour une application qui est totalement différente de l'application spécifique de l'invention, puisqu'elle vise à augmenter la résistance à la corrosion des surfaces des matériaux en question. L'idée est de diminuer la mouillabilité de la surface. A ce propos, on peut indiquer qu'il n'y a pas de corrélation évidente et simple, en raison de la nature du sang, entre la mouillabilité et la thrombogénéicité. Par ailleurs, on préconise, selon ce brevet américain, d'utiliser de l'hélium dans le gaz de traitement. L'effet est d' augmenter le caractère hydrophile. Selon l'invention, on diminue, dans une certaine mesure, le caractère hydrophile de la surface par le remplacement d'une partie des atomes d'hydrogène par des atomes de fluor ou des groupements $CF_2$ ou $CF_3$.

Ces conditions de traitement dont il convient de signaler qu'elles seront mises au point en fonction des polymères traités, signifient notamment:
- une durée de traitement allant de quelques secondes à
- une dizaine de minutes ;
- une puissance d'émission allant de 0, 1 Watt à 2 Watts par litre de capacité de réacteur.

Par ailleurs, le traitement par plasma selon l'invention peut être effectué notamment selon les techniques connues suivantes :
- à pression ambiante, selon la méthode dite décharge Couronne ou encore décharge Corona, cette méthode s'appliquant aux objets plats de faibles épaisseurs (quelques millimètres), car elle nécessite de faire passer les objets à traiter entre deux électrodes créant un arc électrique ;

- à pression réduite, de l'order de 10-1000 Pa, notamment de l'ordre de 66,66 Pa, selon la méthode des plasmas dits "froids". Cette technique permet de traiter des objets volumineux ayant toutes sortes de relief, et convient même pour des substrats poreux. Le traitement est considéré comme suffisamment homogène.

Le procédé selon l'invention consiste donc à exposer les objets à traiter, dans des conditions spécifiques, à l'action de molécules non polymérisables se trouvant à l'état gazeux et comportant des atomes de fluor, excitées par des décharges électromagnétiques, à des fréquences se situant, selon les conceptions d'appareillages, dans les gammes des kHz, des MHz et des GHz. Il convient d'insister sur le caractère ménagé des conditions de traitement, lequel, s'il était réalisé dans des conditions sévères, procurerait, non seulement des résultats médiocres, mais encore des résultats rendant l'objet traité inférieur du point de vue des qualités recherchées à objet non traité. On présentera ci-après, à l'appui de cette caractéristique essentielle du procédé de l'invention, les résultats expérimentaux correspondants.

Les molécules fluorées qui peuvent être utilisées dans la présente invention sont notamment les suivants :

- le fluor ($F_2$)
- le tétrafluorométhane ($CF_4$)
- le chlorotrifluorométhane ($CClF_3$)
- le bromotrifluorométhane ($CBrF_3$)
- le dichlorodifluorométhane ($CCl_2F_2$)
- le dichlorofluorométhane ($CHCl_2F$)
- le trichlorofluorométhane ($CCl_3F$)
- le chlorodifluorométhane ($CHClF_2$)
- le fluorure d'hydrogène ($HF$)
- le trifluorométhane ($CHF_3$)
- le tétrafluorure de soufre ($SF_4$)
- l'hexafluorure de soufre ($SF_6$)
- le trifluorure de bore ($BF_3$)
- le trichlorotrifluoréthane ($C_2Cl_3F_3$)
- le dichlorotétrafluoréthane ($C_2Cl_2F_4$)
- l'hexafluoréthane ($C_2F_6$)
- le tétrafluorosilane ($SiF_4$)
- le chloropentafluoréthane ($C_2ClF_5$)
- le difluorochloréthane ($C_2H_3ClF_2$)
- le difluoréthane ($C_2H_4F_2$)
- le fluorure de méthyle ($CH_3F$)
- le trifluorure d'azote ($NF_3$)
- le pentafluorure de phosphore ($PF_5$).

Ces molécules fluorées peuvent être utilisées seules ou en mélange entre elles, ou encore mélangées avec des gaz non fluorés, tels que des gaz rares (argon, hélium) ou des gaz mono ou polyatomiques, par exemple, l'azote.

A l'issue de l'exposition dans le gaz activé par les décharges électromagnétiques, aucun post-traitement n'est utile. L'objet traité, en une seule étape, peut, en règle générale, être stérilisé par les méthodes habituelles : vapeur, oxyde d'éthylène, rayonnements, sans risquer de détérioration de l'objet modifié.

Le procédé selon l'invention permet donc de modifier les objets sur une épaisseur comprise entre 1 nm et 1 $\mu$m environ : le reste de la matière n'est pas atteint, ce qui permet de conserver les propriétés massiques et, notamment, les propriétés mécaniques, la souplesse et la résistance à la fatigue ; des essais de précoagulation ont montré que les implants traités fixent très peu de globules rouges, contrairement au témoin qui présente des amas, surtout dans les interstices des tissus, la conséquence étant qu'un traitement de précoagulation n'est plus nécessaire.

Pour mieux illustrer l'objet de la présente invention, on va en décrire maintenant, à titre d'exemples purement illustratifs et non limitatifs, plusieurs modes de mise en oeuvre.

A Réalisation de cathéters en polyéthylène

1 - Mode opératoire général et tests

Un cathéter en polyéthylène pur, d'un diamètre intérieur de 1 mm et d'une longueur de 1 mètre, est traité par la méthode des plasmas froids, avec du tétrafluorométhane, à une pression donnée, sous une puissance donnée, pendant un temps donné. La machine émet à une fréquence de 13,56 MHz et présente un volume de 15 litres environ.

On a réalisé plusieurs essais, en faisant varier les conditions de traitement. Chaque essai a été réalisé en triplicate.

On a mesuré, pour chacun des échantillons traités, le temps de perméabilité et le taux de fibrinopeptide, selon le test de Dudley modifié Eloy tel que rapporté dans l'ouvrage «Les matières plastiques à usage pharmaceutique et médico-chirurgical. E. POSTAIRE. Tome 1, pages 208-209. Editions médicales Internationales (février 1987)». Après les tests précédents, on a effectué un examen des échantillons au microscope électronique à balayage.

Le temps de perméabilité, ou temps de thrombose, correspond au temps d'écoulement du sang, à l'intérieur du cathéter, avant obstruction par formation d'un caillot. Il est recherché des temps les plus longs possibles.

Le taux de fibrinopeptide (FpA) est obtenu par coagulation de la fibrinogène en fibrine et fibrinopeptide. Il est nécessaire que le chiffre soit le plus petit possible.

2 - Résultats

Les résultats sont rapportés dans le tableau I suivant :

TABLEAU 1

| Exemples | Conditions du traitement | | | Temps de perméabilité (mn) | FpA | | Microscopie électronique après tests précédents |
|---|---|---|---|---|---|---|---|
| | Puissance (W) | Temps (mn) | Pression (Pa) | | 6ème minute (ng/ml) | 9ème minute (ng/ml) | |
| Témoin | - | - | - | 13,02 | 42,8 | > 172 | Placards de plaquettes |
| Comparatif A | 0 | 10 | 53,33 | 11,76 | 42,8 | > 115 | Peu d'agrégats plaquettaires |
| 1 | 20 | 2 | 66,66 | 18,76 | - | 48 | " |
| 2 | 15 | 10 | 53,33 | 7,30 | 200 | - | " |
| Comparatif B | 50 | 1 | 53,33 | 8,65 | > 850 | > 3420 | Tapis plaquettaire |

6

Ces résultats montrent la nette supériorité du matériau des exemples 1 et 2 (traitement ménagé), sur le matériau témoin (n'ayant subi aucun traitement) et sur les matériaux des exemples comparatifs (notamment B, traitement sévère). On observe ainsi, dans le premier cas, une augmentation du temps de perméabilité et une diminution du FpA, c'est-à-dire du caractère thrombogène, et dans les autres cas, une diminution du temps de perméabilité et augmentation de FpA, c'est-à-dire du caractère thrombogène.

On a ainsi mis en évidence que les traitement doux sont bénéfiques pour l'hémocompatibilité et la thrombogénéité tandis que des traitements sévères sont néfastes.

B Réalisation de prothèses cardiovasculaires en poly(éthylène téréphtalate)

1 - Mode opératoire général

Une prothèse cardiovasculaire en poly(éthylène téréphtalate), tricotée, double velours greffé, d'un diamètre de 12 mm, est traitée par la méthode des plasmas froids, avec du tétrafluorométhane, à une pression donnée, sous une puissance donnée, pendant un temps donné. La machine émet à une fréquence de 13,56 MHz et présente un volume de 15 litres environ.

On a réalisé plusieurs essais, en faisant varier les conditions de traitement. Chaque essai a été réalisé en triplicate.

On prépare ensuite des éprouvettes de la façon suivante :

Les prothèses sont coupées longitudinalement selon une génératrice. La surface est ensuite développée puis étirée transversalement. Les éprouvettes sont alors découpées parallèlement aux spires à l'aide d'un emporte-pièce qui assure une largeur constante de 4 mm.

Les essais de traction ont été réalisés dans les conditions suivantes :

Vitesse de traction : 20 mm/mn ;

écartement initial entre mâchoires pneumatiques

réglé au pied à coulisse : 20 mm.

2 - Résultats

On a déterminé :
- la contrainte à la rupture en daN/cm$^2$ ;
- l'allongement à la rupture en % ; et
- la pente de la tangente à l'origine qui représente le module d'Young en daN/cm$^2$.

Le tableau II rassemble les résultats obtenus.

| Ex | Traitement plasma | | | Contrainte rupture (daN/cm$^2$) | Allongement rupture (%) | Module d'Young transversal (daN/cm$^2$) |
|---|---|---|---|---|---|---|
| | Puissance (W) | Temps | Pression (Pa) | | | |
| Témoin | - | - | - | 84,8 | 189 | 78,8 |
| Ex. 3 | 50 | 3mn | 40 | 85,6 | 185 | 78,2 |
| Ex. Comp. C | 50 | 1 h | " | 82 | 151,3 | 99 |

L'examen des résultats montre que le traitement plasma de 3 mn (exemple 3) conduit à des valeurs de module tout à fait similaires au témoin. Les valeurs de résistance rupture et d'allongement rupture ne sont pas statistiquement différentes. En revanche, une exposition au plasma de 1 h (exemple comparatif C) a tendance à rigidifier le matériau, puisque le module passe de 79 daN/cm$^2$ pour le témoin à 100 daN/cm$^2$. A une résistance à la rupture sensiblement égale, l'allongement à la rupture diminue de façon significative.

**Revendications**

**1.** Article destiné à être utilisé dans des applications nécessitant qu'il présente une forte hémocompatibilité et une faible thrombogénéicité au moins sur la partie de sa surface (dite surface exposée) susceptible de venir en contact avec du sang, ledit article étant constitué d'un matériau polymère d'origine naturelle ou synthétique contenant des atomes d'hydrogène, caractérisé par le fait que des atomes d'hydrogène présents sur sa surface exposée ont été remplacés par des atomes de fluor ou

des groupements $CF_2$ ou $CF_3$, de telle sorte que le fluor total représente au plus 10% des atomes présents sur cette surface.

2. Article selon le revendication 1, caractérisé par le fait qu'il consiste en un objet venu du moulage ou de l'extrusion pour constituer notamment un cathéter, une seringue et similaires.

3. Article selon la revendication 2, caractérisé par le fait que le matériau qui le constitue est le polyéthylène ou le poly(chlorure de vinyle).

4. Article selon la revendication 1, caractérisé par le fait qu'il est fait d'un matériau tissé ou tricoté, pour constituer notamment une prothèse vasculaire ou valvulaire cardiaque.

5. Article selon la revendication 4, caractérisé par le fait que le matériau qui le constitue est le poly-(éthylène téréphtalate)

6. Procédé d'obtention de l'article tel que défini à l'une des revendications 1 à 5, caractérisé par le fait qu'il consiste à effectuer un traitement de surface dudit article se trouvant dans sa forme appropriée pour son utilisation, par un plasma de molécules fluorées non polymérisables, dans des conditions ménagées.

7. Procédé selon la revendication 6, caractérisé par le fait que l'on réalise le traitement de surface pendant une durée allant de quelques secondes à une dizaine de minutes.

8. Procédé selon l'une des revendications 6 et 7, caractérisé par le fait que l'on réalise le traitement de surface à une puissance d'émission allant de 0, 1 Watt à 2 Watts par litre de capacité de réacteur.

9. Procédé selon l'une des revendications 6 à 8, caractérisé par le fait que l'on réalise le traitement à une pression de l'ordre de 10-1000 Pa.

10. Procédé selon l'une des revendications 6 à 8, caractérisé par le fait l'on effectue le traitement à la pression ambiante, selon la méthode dite décharge Couronne ou Corona

11. Procédé selon l'une des revendications 6 à 10, caractérisé par le fait qu'on choisit les molécules fluorées du plasma parmi le fluor, le tétrafluorométhane, le chlorotrifluorométhane, le bromotrifluorométhane, le dichlorodifluorométhane, le dichlorofluorométhane, le trichlorofluorométhane, le chlorodifluorométhane, le fluorure d'hydrogène, le trifluorométhane, le tétrafluorure de soufre, l'hexafluorure de soufre, le trifluorure de bore, le trichlorotrifluoréthane, le dichlorotétrafluoréthane, l'hexafluoréthane, le tétrafluorosilane, le chloropentafluoréthane, le difluorochloréthane, le difluoréthane, le fluorure de méthyle, le trifluorure d'azote et le pentafluorure de phosphore.

12. Procédé selon la revendication 11, caractérisé par le fait qu'on utilise, comme molécules fluorées, des molécules de tétrafluorométhane.

**Claims**

1. An article for use in applications where it needs to have a high hemocompatibility and a low thrombogenicity, at least over that part of its surface (called exposed surface) which may come into contact with blood, the said article consisting of a polymeric material of natural or synthetic origin containing hydrogen atoms, characterized in that hydrogen atoms present on its exposed surface have been replaced with fluorine atoms or $CF_2$ or $CF_3$ groups, so that the total fluorine represents at most 10% of the atoms present on this surface.

2. An article according to claim 1, characterized in that it consists of a molded or extruded object, especially for producing a catheter, a syringe or the like.

3. An article according to claim 2, characterized in that the material of which it is made is polyethylene or polyvinyl chloride.

4. An article according to claim 1, characterized in that it is made of a woven or knitted material, especially for producing a vascular prosthesis or heart valve prosthesis.

5. An article according to claim 4, characterized in that the material of which it is made is polyethylene terephthalate.

6. A process for the manufacture of the article as defined in any one of claims 1 to 5, characterized in that it consists in carrying out a surface treatment on the said article in the form appropriate for its use, with a plasma of non-polymerizable fluorinated molecules, under controlled conditions.

7. The process according to claim 6, characterized in that the surface treatment is carried out for a time ranging form a few seconds to about ten minutes.

8. The process according to claim 6 or claim 7, characterized in that the surface treatment is carried out at an emission power ranging from 0.1 Watt to 2 Watts per liter of reactor capacity.

9. The process according to any one of claims 6 to 8, characterized in that the treat is carried out at a pressure of the order of 10-1000 Pa.

10. The process according to any one of claims 6 to 8, characterized in that the treatment is carried out at ambient pressure by the so-called corona discharge method.

11. The process according to any one of claims 6 to 10, characterized in that the fluorinated molecules of the plasma are selected from fluorine, carbon tetrafluoride, chlorotrifluoromethane, bromotrifluoromethane, dichlorodifluoromethane, dichlorofluoromethane, trichlorofluoromethane, chlorodifluoromethane, hydrogen fluoride, trifluoromethane, sulfur tetrafluoride, sulfur hexafluoride, boron trifluoride, trichlorotrifluoroethane, dichlorotetrafluoroethane, hexafluoroethane, tetrafluorosilane, chloropentafluoroethane, difluorochloroethane, difluoroethane, methyl fluoride, nitrogen trifluoride and phosphorus pentafluoride.

12. The process according to claim 11, characterized in that carbon tetrafluoride molecules are used as the fluorinated molecules.

**Patentansprüche**

1. Erzeugnis, das für Anwendungen bestimmt ist, für die es eine verbesserte Blutverträglichkeit und eine geringere Thrombogenizität auf wenigstens dem Teil seiner Oberfläche (als freiliegende Oberfläche bezeichnet), die mit Blut in Kontakt kommen kann, aufweist, wobei das Erzeugnis aus polymerem Material natürlichen oder künstlichen Ursprungs ist und Wasserstoffatome enthält, dadurch gekennzeichnet, daß die auf der freiliegenden Oberfläche befindlichen Wasserstoffatome durch Fluoratome oder $CF_2$- oder $CF_3$-Gruppen derart ersetzt sind, daß das Fluor insgesamt höchstens 10% der auf dieser Oberfläche befindlichen Atome ausmacht.

2. Erzeugnis nach Anspruch 1, dadurch gekennzeichnet, daß es aus einem durch Formspritzen oder durch Extrusion erhaltenen Gegenstand besteht, der insbesondere ein Katheter, eine Spritze oder ähnliches bildet.

3. Erzeugnis nach Anspruch 2, dadurch gekennzeichnet, daß das Material, aus dem es besteht, Polyäthylen oder Polyvinylchlorid ist.

4. Erzeugnis nach Anspruch 1, dadurch gekennzeichnet, daß es aus gewirktem oder gestricktem Material gefertigt ist, um insbesondere eine Gefäß- oder eine Herzklappenprothese zu bilden.

5. Erzeugnis nach Anspruch 4, dadurch gekennzeichnet, daß das Material, aus dem es besteht, Polyäthylenterephtalat ist.

6. Verfahren zum Herstellen eines Erzeugnisses nach einem der Ansprüche 1 bis 5, gekennzeichnet durch das Ausführen einer Oberflächenbehandlung des sich in einer verwendungsgerechten Form befindlichen Erzeugnisses durch ein Plasma aus fluorierten, nicht polymerisierbaren Molekülen unter geeigneten Bedingungen.

7. Verfahren nach Anspruch 6, dadurch gekennzeichnet, daß die Oberflächenbehandlung über eine Dauer von einigen Sekunden bis zu etwa zehn Minuten durchgeführt wird.

8. Verfahren nach einem der Ansprüche 6 oder 7, dadurch gekennzeichnet, daß die Oberflächenbehandlung mit einer Leistung von 0,1 bis 2 Watt pro Liter Reaktionsgefäßkapazität ausgeführt wird.

9. Verfahren nach einem der Ansprüche 6 bis 8, dadurch gekennzeichnet, daß die Behandlung bei einem Druck von etwa 10 bis 1000 Pa durchgeführt wird.

10. Verfahren nach einem der Ansprüche 6 bis 8, dadurch gekennzeichnet, daß die Behandlung bei Umgebungsdruck nach dem Prinzip der Koronaentladung durchgeführt wird.

11. Verfahren nach einem der Ansprüche 6 bis 10, dadurch gekennzeichnet, daß die fluorierten Plasmamoleküle aus folgenden Stoffen ausgewählt werden: Fluor, Tetrafluormethan, Chlortrifluormethan,Bromtrifluormethan, Dichlordifluormethan, Dichlorfluormethan, Trichlorfluormethan, Chlordifluormethan, Fluorwasserstoff, Trifluormethan, Tetrafluorschwefel, Hexafluorschwefel, Bortrifluorid, Trichlortrifluoräthan, Dichlortetrafluoräthan, Hexafluoräthan, Tetrafluorsilan, Chlorpentafluoräthan, Difluorchloräthan, Difluoräthan, Methylfluorid, Stickstofftrifluorid und Phosphorpentafluorid.

12. Verfahren nach Anspruch 11, dadurch gekennzeichnet, daß als fluorierte Moleküle Tetrafluormethanmoleküle verwendet werden.